# EUROPEAN PATENT APPLICATION

(11) **EP 1 064 938 A1**
(43) Date of publication of application: **03.01.2001**
(21) Application number: 99401606.1
(22) Date of filing: 28.06.1999
(51) Int. Cl.: A61K 9/50

(54) **Pharmaceutical dosage forms for controlled release producing at least a timed pulse**

(71) Applicant: Sanofi-Synthélabo, 75013 Paris (FR)
(72) Inventor: André, Frédéric, 92160 Antony (FR); Lewis, Gareth, 91410 Dourdan (FR); Mignonneau, Jérôme, 49350 Saint Clément des Levées (FR); Ribardière, Agnès, 92400 Courbevoie (FR)
(74) Representative: Thouret-Lemaitre, Elisabeth

(57) **Abstract**

The present invention relates to delayed release coated cores comprising an active substance in their core and a polymer coating comprising at least one or more ammonio methacrylate copolymer, characterised in that the core comprises at least a surfactant ; to monolithic or multiparticulate pharmaceutical dosage forms comprising such delayed release coated cores and to their method of manufacture.

## Description

The present invention relates to controlled release dosage forms producing at least a timed pulse, that is a rapid and complete controlled release of a pharmaceutical substance a fixed time after administration.

Most pharmaceutically active substances administrated orally are given as conventional immediate release or rapid release forms. Thus, provided drug release and absorption are rapid, the concentration time profile of the active substance in the blood or other body compartment depends on the kinetics of elimination of the molecule from the body, and on the distribution and kinetics of distribution in different body compartments and tissues.
This limits the time the drug spends in the body components and thus the time of action of the drug. For this reason, in order to increase the residence time of the drug, prolonged release dosage forms are used, allowing less frequent dosing. In the past, it has often been considered for most drugs that there is an optimum plasma level, and thus the best formulation will be one that gives blood plasma concentration profiles as near constant as possible, and allows reduced dosing frequency.

However such release patterns giving constant plasma levels are not always optimal.
Physiological processes are indeed most of the time not constant over time and circadian rhythms have been shown for almost all bodily functions, as well as symptoms of certain diseases.
For example, myocardial infarction and ischemia and angina pectoris, attacks are more frequent in morning hours 6 - 12 am, and occur particularly in the 4 hours following awaking. Thus it would be preferable in the treatment of these diseases to ensure relatively high blood levels of the drug over that period. For example, an evening administration at 21.00 could then imply an increased release rate about 7-10 hours after administration. Examples of other diseases and symptoms showing a circadian pattern are inflammatory diseases, nocturnal asthma, migraine headache, ulcer, including perforated ulcer, intractable pain and pain from rheumatoid arthritis.

Controlled release dosage forms producing a timed pulse are therefore particularly adapted in the treatment of the here above cited diseases and symptoms thereof. In other words, they can be used for the corresponding chronotherapeutic treatments.

It is also well known that drug release in the form of a pulse rather than a steady slow release may reduce loss by a saturable first-pass effect as in the case of levadopa or propoxyphene. In addition, certain receptors are inactivated by prolonged stimuli, and a pulsed, or on-off delivery can overcome this effect.

As another advantage timed release can allow targeting of a drug to a given site of the gastrointestinal tract, in particular the colon. This depends on the near constant transit time of a pharmaceutical dosage form through the small intestine. A rapid release of the drug in the colon may have advantages in allowing a high local concentration and improved absorption, since absorption of many drugs is much slower and less complete from the colon than from the small intestine, and absorption may become the rate-limiting step rather than release from the dosage form.

It is therefore clear that formulations producing a timed pulse are useful, for example, as described above, for obtaining a non-constant blood plasma concentration profile compatible with and optimal for the therapeutic objective, or for compensating the differences in the rate and extent of absorption in different portions of the gastrointestinal tract, and so obtaining minimally fluctuating blood levels over the entire dosing period.

Dosage forms for controlled release producing at least a timed pulse may also be useful as complementary treatment of an initial treatment. For example, the effect of an initial active substance, which acts rapidly may be suppressed or completed by a second active substance released a fixed time after administration of the dosage form comprising both of the active substances.

Until now, one of the known methods of achieving a timed pulse from a single galenic entity consists in coating a core comprising the active substance with a polymer coating comprising at least one or more methacrylate copolymers containing quaternary ammonium groups. These are referred to as ammonio methacrylate copolymers.

Dosage forms formulated from these here above described coated cores can give sigmoidal release profiles but not real timed pulse profiles. In other words the achieved release rate is often not rapid enough. And another disadvantage of this technique is related to the fact that a large amount of the drug is not released from the coated cores.

The first object of the present invention is then related to a pharmaceutical dosage form for a timed pulse release, whereby the release rate is zero or very low during a fixed time and then the whole of the drug comprised in the dosage form is released rapidly.

Indeed the applicant has found surprisingly that the addition of small quantities of a surfactant into a core comprising the active substance, which is coated with at least one or more ammonio methacrylate copolymer, as described above, give a delayed accelerated pulse, and substantially more complete release of the drug.

The term "particle" in the whole description encompasses all galenic entities variously known as pellets, beads, granules or spheroids.

The core may be a tablet or a particle and the dosage form may be monolithic, that is a single tablet, or multiparticulate, that is either several tablets or a large number of particles. Multiple particles may be within a capsule. Alternatively a large number of particles may be compressed into a tablet which disintegrates in aqueous fluids, releasing the particles.

For reasons of simplicity, in the whole description, the resulting particle or tablet is named "delayed release particle", or "delayed release tablet" or more generally "delayed release coated core".

Thus the present invention, as a first object, provides delayed release coated cores comprising an active substance in their core and a polymer coating comprising at least one or more ammonio methacrylate copolymer, characterised in that the core comprises at least a surfactant.
The present invention also provides monolithic or multiparticulate pharmaceutical dosage forms comprising such delayed release coated cores, producing one unique timed pulse.
The present invention also provides the method of manufacture of the delayed release coated cores and the pharmaceutical dosage forms containing them.

Ammonio methacrylate can be of two types, A and B. These are for example marketed by Röhm Pharma as Eudragit® RS and Eudragit® RL, respectively. Type A, like Eudragit® RS, is relatively impermeable to water and small molecules, and Eudragit® RL is relatively permeable.
According to the invention other polymers and pharmaceutical adjuvants well known to persons with ordinary skill in the art of pharmaceutical formulation may also be incorporated in the coating. The polymers may include cellulosic derivatives such as ethylcellulose or hydroxypropylmethylcellulose (ou hypromellose), and other adjuvants are plastifiers such as diacetylated monoglycerides or triethyl citrate, and antitack agents such as talc.

According to the present invention the additional surfactant is either cationic or amphoteric and/or zwitterionic in nature.

In fact, an additional surfactant diffuses into the polymer coating, and at a given level provokes a sudden change in the film's properties.
Examples of such cationic surfactants are trimethyl-dimyristoyl-ammonium propionate, dimethyl-dioctadecyl-ammonium bromide, trimethyl-cetyl-ammonium bromide (CTAB), dimethyl-didodecyl-ammonium bromide (DDAB(12)), benzalkonium chloride, cetylpyridinium chloride or cetrimide.
Other salts of the above cationic surfactants may equally be employed.

Preferred examples of cationic surfactants are benzalkonium chloride and cetylpyridinium chloride.

Examples of zwitterionic surfactants are the N-alkylbetaines, the C-alkylbetaines, the N-alkylamidobetaines such as cocamidopropylbetain ; the N-alkylglycines and the phosphatidylcholines or lecithins.

The present invention also extends to the use of mixtures of cationic and/or zwitterionic surfactants especially mixtures of the afore mentioned surfactants.

Suitable active substances may be selected from, for example, hormones, polysaccharides, polypeptides, steroids, hypnotics and sedatives, psychic energizers, tranquilizers, anticonvulsants, muscle relaxants, antiparkinson agents, analgesics, anti-inflammatories, muscle contractants, sympathomimetics, polypeptides and proteins capable of eliciting physiological effects, diuretics, lipid regulating agents, antiandrogenic agents, neoplastics, antineoplastics, hypoglycemics, antienteritis agents, and diagnostic agents.

Exemples of active substance useful in this invention include diltiazem, theophylline, felodipine, verapamil clonidine, acebutolol, alprenolol, betaxolol, metoprolol, nadolol, propranolol, timolol, captopril, enalapril, fosinopril, tiapamil, gallopamil, amlodipine, nitrendipine, nisoldipine, nicardipine, felodipine, molsidamine, indomethacin, sulindac, indoprofen, ketoprofen, flurbiprofen, fenbufen, fluprofen, diclofenac, tiaprofenic acid, naproxen, mizolastin, terbutaline, salbutamol, betamethasone, prednisone, methylprednisone, dexamethasone, prednisolone, sumatriptan, naratriptan, cimetidine, ranitidine, famotidine, nizatidine , omeprozole, morphine, fenoprofen, ibuprofen, ketoprofen, alclofenac, mefenamic, alfuzosin, prazosin, tamsulosin, levodopa and methyldopa, their salts and pharmacologically active esters.

In advantageous embodiments, dosage forms may be formulated in order to obtain a timed pulse release independent of the pH. The preferred manner to achieve such a release, in the case of a basic drug is to add a pharmaceutically acceptable organic acid into the dosage form, according to methods known from one skilled in the art. Such dosage forms are preferred.
These pharmaceutically acceptable organic acids can be chosen for example among maleic, tartaric, malic, fumaric, lactic, citric, adipic or succinic acid and their acid salts where these exist, in the form of racemates or isomers, where these exist. According to the invention, acids particularly preferred are tartaric, fumaric, citric, and succinic and their acid salts.

The amount of cationic or zwitterionic surfactant which may be used with the present invention may vary but preferably is between 10 and 50% with respect to the amount of ammonio methacrylate copolymer in the coating.

The dosage forms according to the present invention include capsules, tablets, multicoated tablets, granulates.

Various formulations, not limiting the scope of the present invention, illustrating the first object of the invention, that is pharmaceutical dosage forms producing one unique timed pulse, are described hereafter:
(1) Delayed release particles containing a drug :
   These are particles of dimension for example 0.2 to 2 mm diameter, comprising in addition to the drug at least a cationic surfactant in the core and with a polymer coating comprising at least one or more ammonio methacrylate copolymers.
   The particles may be manufactured by any of the methods well known to one skilled in the art: granulation in a high speed granulator, extrusion followed by spheronisation, gradual coating of a sugar sphere with a mixture comprising the drug etc.
   The particles are coated for delayed release with a coating comprising one or more ammonio methacrylate copolymers. In addition the coating may comprise one or more other polymers impermeable to water and to drug molecules, such as ethylcellulose, cellulose acetate, cellulose acetate butyrate, polyvinyl chloride, polyvinylacetate. The coating may also comprise one or more polymers which are permeable to water, such as hydroxypropylmethyl-cellulose, hydroxyethylcellulose.
   The composition of the mixture and the amount of coating applied is adjusted to allow gradual hydration of the film and a delayed release profile.
   The core may comprise other substances necessary, in particular an organic acid to maintain the pH at the interior of the particle constant.
   The particles may be filled in a unique dosage form as a gelatin capsule.
(2) Delayed release tablets comprising a drug and at least a cationic surfactant in the core and with a polymer coating comprising at least one or more ammonio methacrylate copolymers.
   These are formulated by the methods well known to one skilled in the art.
   In addition to the drug and the cationic surfactant they can comprise inert pharmaceutical excipients, including one or more diluants, for example microcrystalline cellulose, lactose, mannitol, starch ; and may contain other excipients.
   These can include one or more binders, for example hydroxypropylmethylcellulose, ethylcellulose and povidone, lubricants, for example magnesium stearate, glyceryl stearate, and glyceryl behenate, disintegrants, for example crospovidone, sodium starch glycolate and croscarmellose, glidants, for example talc and colloidal silicon dioxide. In particular a pharmaceutically acceptable acid may be added to ensure liberation of the basic active substances independent of the pH of the external medium.
   The tablets can be prepared by compression of a simple mixture or a granulate, followed by coating with a polymer solution.

Minitablets which are also emcompassed in the invention are tablets of dimension 3 mm or less. They can be used for achieving dosage forms for timed pulse release. They can be manufactured using the same components as described above.

The delayed release tablets can be coated with a layer of polymer coating similar to those described for the multiparticulate systems above. However except in the case of the minitablets some modification of the coating may be required because of the difference in surface area of the dosage form.

It is usually necessary to apply a thicker coating on the tablet than on the particles, and thus a higher proportion of water-permeable polymers can be required in the coating composition.

The delayed release tablets or minitablets may be used alone. The minitablets may also been filled into envelopes such as hard gelatine capsules.

Moreover, as a further object, the invention also encompasses all dosage forms comprising delayed release coated cores according to the invention combined together to give a "stepped" release profile or with other galenic entities. These other galenic entities can for example be immediate or sustained release systems.
As described above, these further dosage forms can also be used for example in chronotherapeutic treatments, to overcome the first pass effect, or to improve the absorption according to a given part of the gastrointestinal tract.

The other galenic entities may contain the same active substance as the delayed release entity or a different active substance. Indeed, when comprising two different active substance, dosage forms can for example be formulated in order to obtain the complementary treatment described hereinabove.

In particular an object of the present invention is related to pharmaceutical compositions for timed dual release, whereby a first release pulse occurs immediately and a second release pulse is delayed to a fixed time.

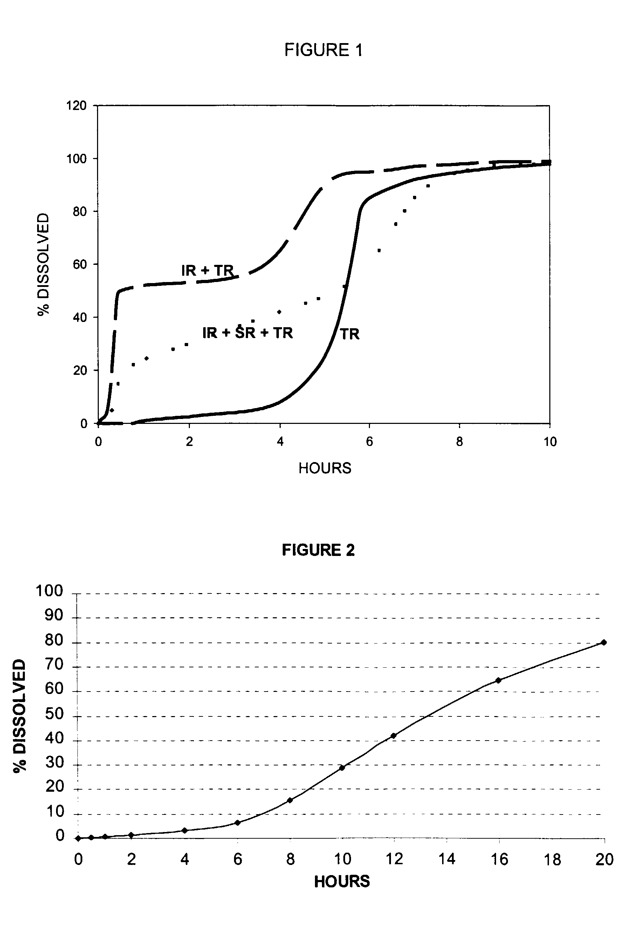

Examples of the different types of profiles which may be obtained by combining formulations according to the invention with other galenic entities are shown in figure 1.

The following formulations illustrate this further object of the invention, that is dosage forms comprising delayed release coated cores according to the invention combined together to give a "stepped" release profile or with other galenic entities :
(1) Capsule comprising the delayed release particles or minitablets according to the invention and an immediate and/or sustained release entities
   The required amount of delayed release particles or minitablets according to the invention are combined with one or both of the following
   (i) immediate release (uncoated) particles or minitablets or an immediate release granulate or powder
   (ii) sustained release particles or minitablets (coated, slow release)
   in hard gelatine capsules of the required size.
   Particles or minitablets with different delayed release profiles may also be combined to give a "stepped" release profile.
(2) A tablet comprising delayed release particles according to the invention imbedded in a rapidly disintegrating matrix.
   The matrix may also comprise the drug substance. Sustained (slow) release particles may be included in addition to the delayed release particles.
   Alternatively the tablet may consist of a mixture of delayed release particles and of immediate release non-coated particles comprising the active substance, imbedded in a matrix free from the drug.
   Alternatively the delayed release particles may be furthermore coated with a layer comprising the drug and other excipients allowing immediate release from that layer, imbedded in a matrix free from the drug.
   Alternatively the delayed release tablet may consist of one or more layers comprising delayed release particles comprising the drug, imbedded in a matrix free from the drug and one or more layers comprising the drug in an immediate release matrix.
   The matrix surrounding the particles should preferably be formulated so that the compression into tablets does not interfere with the integrity of the membrane surrounding the pellets. On contact with fluid the tablet disintegrates, releasing the drug rapidly, from the matrix, or the immediate release pellets, or from the immediate release particle coating, or from the immediate release layer, and then, after a fixed interval of time, releases the drug from the delayed release particles.
   In the case of a basic drug the particle may be formulated with a pharmaceutically acceptable organic acid so as to maintain the micro-pH of the particle during release in the neutral pH conditions.
   The matrix can consist of inert pharmaceutical substances such as well known to one skilled in the art of pharmaceutical formulation. In particular the matrix can include one or more diluants such as microcrystalline cellulose, lactose, mannitol, starch and one or more disintegrants, for example crospovidone, sodium starch glycolate and croscarmellose. Other excipients may also be included, lubricants, for example magnesium stearate, glyceryl stearate, and glyceryl behenate, binders, for example hydroxypropylmethylcellulose, ethylcellulose and povidone, glidants, for example talc and colloidal silicon dioxide.
(3) Capsule comprising one or more immediate release tablets and one or more delayed release tablets.
   The delayed release tablets are prepared as described above. Immediate release tablets can be made exactly the same way, except they are uncoated, do not require a cationic surfactant and do not normally require addition of an acid. Instead of or as well as the immediate release tablet, one or more sustained (slow) release tablets may be included in the formulation.
(4) Multicoated tablets
   Delayed release tablets are prepared as described above and press coated with an immediate release soluble or disintegrable coating.

### List of figures:

Figure 1 shows examples *in vitro* release profiles, where the full curve shows a delayed release profile (TR), the dashed curve shows the combination of an immediate release with a delayed release profile (IR + TR), and the dotted curve shows the combination of both immediate release and sustained release profiles with a delayed release profile (IR + SR + TR).

Figure 2 shows an *in vitro* dissolution profile of the coated pellets containing alfuzosin hydrochloride of example 1.

Figure 3 shows an *in vitro* dissolution profile of the coated pellets containing alfuzosin hydrochloride of comparative example 1.

Figure 4 shows an *in vitro* dissolution profile of the coated pellets containing alfuzosin hydrochloride of example 2.

Figure 5 shows an *in vitro* dissolution profile of the coated pellets containing alfuzosin hydrochloride of example 3.

Figure 6 shows an *in vitro* dissolution profile of the coated pellets containing alfuzosin hydrochloride of comparative example 3.

The examples which follow illustrate the invention without limiting it:

### Example 1: Capsules containing alfuzosine hydrochloride and cetylpyridinium chloride - slow release after a long interval

3325 g of non-pareil beads 16/18 mesh were loaded with alfuzosin hydrochloride by coating in a GPCG3 fluid bed coater-dryer with a suspension of the following condition

| | | |
|---|---|---|
| alfuzosin hydrochloride | 5.0 % | 87.5 g |
| Polyvinyl alcohol¹ | 5.0 % | 87.5 g |
| purified water | 90.0 % | 1575 g |

| | | |
|---|---|---|
| ¹ Mowiol 5-88® commercialised by Chimidis Hoechst | | |

1100 g of these alfuzosin-coated beads were then coated in a GPCG1 fluid bed coater-dryer using a suspension of the following composition:

| | | |
|---|---|---|
| cetylpyridinium chloride | 4.3 % | 43.4 g |
| succinic acid | 4.7 % | 46.9 g |
| hydroxypropylmethylcellulose² | 5.9 % | 59.0 g |
| purified water | 42.5 % | 425.0 g |
| isopropanol | 42.5 % | 425.0 g |

| | | |
|---|---|---|
| ²Pharmacoat 603® commercialised by Shin-Etsu | | |

Finally 1000 g of beads above described were coated using a polymer solution of the following composition:

| | | |
|---|---|---|
| ammonio methacrylate copolymer Type B ³ | 5.1 % | 119.0 g |
| ammonio methacrylate copolymer Type A ⁴ | 0.3 % | 7.0 g |
| acetylated monoglycerides⁵ | 0.6 % | 14.0 g |
| isopropanol | 56.4 % | 1316.0 g |
| acetone | 37.6 % | 877.3 g |

| | | |
|---|---|---|
| ³ Eudragit® RS100 commercialised by Röhm Pharma | | |
| ⁴ Eudragit® RL100 commercialised by Röhm Pharma | | |
| ⁵ Eastman 9-45 commercialised by Eastman | | |

The dissolution of the beads was measured using the method described in the European pharmacopoeia with the rotating paddle apparatus, at a stirring speed of 100 rpm. Dissolution medium was 500 mL, 0.01M hydrochloric acid at 37°C + 0.5°C. The amount of alfuzosine dissolved was measured by UV spectrophotometry at 330 nm. The dissolution curve obtained is shown in figure 2.

### Comparative example 1: Capsules containing alfuzosine hydrochloride (without cetylpyridinium chloride)

1100 g of the alfuzosin-coated beads, prepared as described in example 1 were coated using a suspension of the following composition :

| | | |
|---|---|---|
| succinic acid | 7.0 % | 46.2 g |
| hydroxypropylmethylcellulose¹ | 8.8 % | 58.3 g |
| purified water | 42.1 % | 277.9 g |
| isopropanol | 42.1 % | 277.9 g |

| | | |
|---|---|---|
| ¹ Pharmacoat 603® commercialised by Shin-Etsu | | |

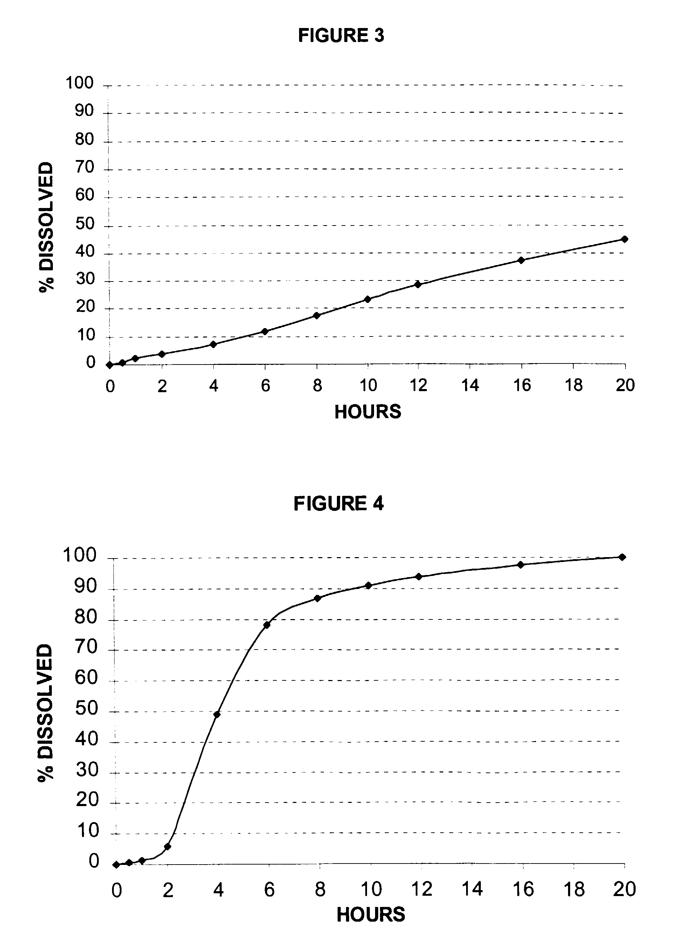

Finally 1000 g of beads above described were coated using a polymer solution as described in example 1
The dissolution profil of the pellets was determined. The dissolution method was that described in example 1. The dissolution curve obtained is shown in figure 3.

### Example 2 : Coated pellets

Delayed release pellets containing alfuzosin hydrochloride, tartaric acid and cetylpyridinium chloride as cationic surfactant

1000 g of nonpareil beads 16/18 mesh were coated using a suspension with the following composition,

| | | |
|---|---|---|
| tartaric acid | 6.0 % | 78.0 g |
| hydroxypropylmethylcellulose ¹ | 4.0 % | 53.0 g |
| cetylpyridinium chloride | 3.0 % | 39.0 g |
| triethyl citrate | 1.4 % | 18.2 g |
| purified water | 43.8 % | 557 g |
| isopropanol | 43.8 % | 557 g |

| | | |
|---|---|---|
| ¹ Pharmacoat 603® commercialised by Shin-Etsu | | |

The pellets were then loaded with alfuzosin hydrochloride by coating with the following solution, in a GPCG1 fluid bed coater-dryer:

| | | |
|---|---|---|
| alfuzosin hydrochloride | 8.3 % | 78 g |
| povidone K30 ² | 8.3 % | 78 g |
| ethanol | 83.4 % | 784 g |

| | | |
|---|---|---|
| ² Kollidon® commercialized by BASF | | |

Finally 1000 g of the pellets were coated using a polymer solution of the following composition :

| | | |
|---|---|---|
| ammonio methacrylate copolymer Type B ³ | 11.40 % | 83.4 g |
| ammonio methacrylate copolymer Type A ⁴ | 0.93 % | 6.8 g |
| triethyl citrate | 1.37 % | 10.0 g |
| isopropanol | 51.80 % | 379.0 g |
| acetone | 34.50 % | 252.0 g |

| | | |
|---|---|---|
| ³ Eudragit® RS100 commercialised by Röhm Pharma | | |
| ⁴ Eudragit® RL100 commercialised by Röhm Pharma | | |

The dissolution profile of the pellets in 0.01 M hydrochloric acid was measured using the method described in example 1. The dissolution curve obtained is shown in figure 4.

### Example 3 : Coated pellets :

Delayed release pellets containing alfuzosin hydrochloride, succinic acid and cocamidopropylbetain as a zwitterionic surfactant

1000 g of nonpareil beads 16/18 mesh were coated using a suspension with the following composition,

| | | |
|---|---|---|
| succinic acid | 5.63 % | 78.0 g |
| hydroxypropylmethylcellulose ¹ | 3.82 % | 53.0 g |
| cocamidopropylbetain ² | 2.81 % | 39.0 g |
| purified water | 43.87 % | 608 g |
| isopropanol | 43.87 % | 608 g |

| | | |
|---|---|---|
| ¹ Pharmacoat 603® commercialised by Shin-Etsu | | |
| ² Amonyl® 380LC commercialised by Seppic | | |

The pellets were then loaded with alfuzosin hydrochloride as described in example 2

Finally 1000 g of the pellets were coated using a polymer solution of the following composition :

| | | |
|---|---|---|
| ammonio methacrylate copolymer Type B ³ | 11.40 % | 208.5 g |
| ammonio methacrylate copolymer Type A ⁴ | 0.93 % | 17 g |
| triethyl citrate | 1.37 % | 25 g |
| isopropanol | 51.80 % | 947.5 g |
| acetone | 34.50 % | 630 g |

| | | |
|---|---|---|
| ³ Eudragit® RS100 commercialised by Röhm Pharma | | |
| ⁴ Eudragit® RL100 commercialised by Röhm Pharma | | |

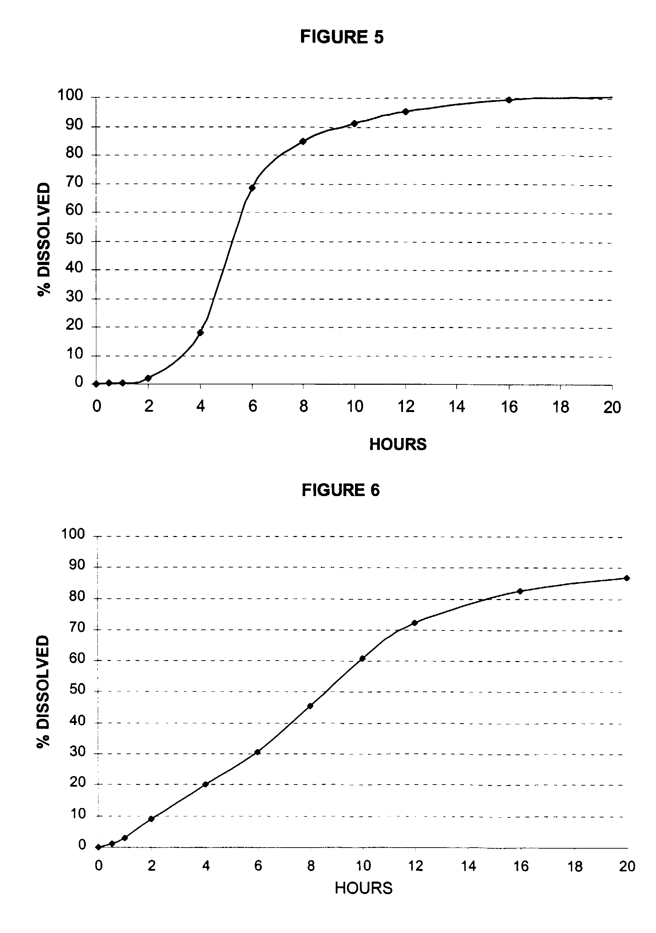

After drying in a ventilated oven, at 30°C for 24 h the dissolution profile of the pellets in 0.01 M hydrochloric acid was measured using the method described in example 1. It is shown in figure 5.

### Comparative example 3 : coated pellets without surfactant

1000 g of non-pareil beads 16/18 mesh were coated using a suspension with the following composition

| | | |
|---|---|---|
| succinic acid | 5.99 % | 78.0 g |
| hydroxypropylmethylcellulose ¹ | 4.07 % | 53.0 g |
| purified water | 44.97 % | 585.5 g |
| isopropanol | 44.97 % | 585.5 g |

| | | |
|---|---|---|
| ¹ Pharmacoat 603® commercialised by Shin-Etsu | | |

The beads were then loaded with alfuzosin hydrochloride according to example 1 and finally coated with polymer using the same methods and composition as described in example 3. The dissolution profiles of the pellets were measured as described in example 1. They are shown in figure 6.

## Claims

1. A delayed release coated core comprising an active substance in its core and a polymer coating comprising at least one or more ammonio methacrylate copolymers, characterised in that the core comprises at least one ore more surfactants.

2. A delayed release coated core according to claim 1, characterised in that the surfactants are cationic or zwitterionic in nature.

3. A delayed release coated core according to claim 1 or 2, characterised in that the ammonio methacrylate copolymers are of type A or B.

4. A delayed release coated core according to anyone of claim 1 to 3, characterised in that the cationic surfactants are chosen among trimethyl-dimyristoyl-ammonium propionate, dimethyl-dioctadecyl-ammonium bromide, trimethyl-cetyl-ammonium bromide, dimethyl-didodecyl-ammonium bromide, benzalkonium chloride, cetylpyridinium chloride and cetrimide.

5. A delayed release coated core according to anyone of claim 1 to 3, characterised in that the zwitterionic surfactants are chosen among N-alkylbetaines, C-alkylbetaines, N-alkylamidobetaines, N-alkylglycines, phosphatidylcholines and lecithins.

6. A delayed release coated core according to claim 5, characterised in that the zwitterionic surfactant is cocamidopropylbetain.

7. A delayed release coated core according to anyone of claim 1 to 6, characterised in that the active substance is chosen among diltazem, theophylline, felodipine, verapamil clonidine, acebutolol, alprenolol, betaxolol, metoprolol, nadolol, propranolol, timolol, captopril, enalapril, fosinopril, tiapamil, gallopamil, amlodipine, nitrendipine, nisoldipine, nicardipine, felodipine, molsidamine, indomethacin, sulindac, indoprofen, ketoprofen, flurbiprofen, fenbufen, fluprofen, diclofenac, tiaprofenic acid, naproxen, mizolastin, terbutaline, salbutamol, betamethasone, prednisone, methylprednisone, dexamethasone, prednisolone, sumatriptan, naratriptan, cimetidine, ranitidine, famotidine, nizatidine , omeprozole, morphine, fenoprofen, ibuprofen, ketoprofen, alclofenac, mefenamic, alfuzosin, prazosin, tamsulosin, levodopa and methyldopa, their salts and pharmacologically active esters.

8. A delayed release coated core according to anyone of claim 1 to 7, characterised in that it is a particle, pellet, bead, granule or spheroid, of a diameter comprised between 0.3 and 3 mm.

9. A delayed release coated core according to anyone of claim 1 to 7, characterised in that it is a tablet.

10. A delayed release coated core according to anyone of claim 1 to 7, characterised in that it is a minitablet.

11. A pharmaceutical dosage form comprising at least a delayed release coated core according to anyone of claims 1 to 10.

12. A pharmaceutical dosage form according to claim 11, characterised in that it takes the form of a tablet, a multilayer tablet, a multicoated tablet or a capsule.

13. A pharmaceutical dosage form according to claim 11 or 12, characterised in that coated cores of differing delayed release times are combined together to give "stepped" release profile.

14. A pharmaceutical dosage form according to claim 11 or 12, characterised in that the release coated core(s) is/are combined with other galenic entitie(s), which release is immediate or sustained.

15. A pharmaceutical dosage form according to claim 14, characterised in that the other galenic entitie(s) contain(s) a different active substance as in the release coated core(s).

16. A pharmaceutical dosage form according to claim 14, characterised in that a first release pulse occurs immediately and a second release pulse is delayed to a fixed time.

17. A capsule according to claim 14, characterised in that it comprises the delayed release coated cores according to claim 8 or 10 and an immediate and/or sustained release entity chosen alternatively among
(i) immediate release particles or minitablets or an immediate release granulate or powder,
(ii) controlled release particles or minitablets.

18. A tablet according to claim 14, characterised in that it comprises the delayed release coated cores according to claim 8 imbedded in a rapidly desintegrating matrix and alternatively in that
(i) the matrix is free of the active substance,
(ii) the matrix also comprises the active substance,
(iii) sustained release particles are mixed to the delayed release particles,
(iv) immediate release particles are mixed with the delayed release coated particles,
(v) the delayed release particles are further coated with a layer comprising the active substance, allowing an immediate release,
(vi) the tablet consists of one or more layers comprising the delayed release particles in the rapidly desintegreting matrix and of one or more layers comprising the active substance in an immediate release matrix.

19. Capsule according to claim 14, characterised in that it comprises one or more immediate release tablets and one or more delayed release tablets according to claim 9.

20. Multicoated tablets according to claim 14, characterised in that the tablet is coated with an immediate release soluble or disintegrable coating.
